# EUROPEAN PATENT SPECIFICATION

(11) **EP 2 553 459 B1**
(45) Date of publication and mention of the grant of the patent: **04.03.2015**
(21) Application number: 11712659.9
(22) Date of filing: 31.03.2011
(51) Int. Cl.: G01N 33/68

(54) **BIOMARKERS FOR SCHIZOPHRENIA**
BIOMARKER FÜR SCHIZOPHRENIE
MARQUEURS BIOLOGIQUES

(30) Priority: 31.03.2010 GB 201005456
(43) Date of publication of application: 06.02.2013
(73) Proprietor: Cambridge Enterprise Ltd., Cambridge Cambridgeshire CB2 1TN (GB)
(72) Inventor: BAHN, Sabine, Cambridge Cambridgeshire CB2 1QT (GB); GUEST, Paul, Cambridge Cambridgeshire CB2 1QT (GB); HARRIS, Laura, Cambridge Cambridgeshire CB2 1QT (GB); LEVIN, Yishai, Hadera 38523 (IL); KRISHNAMURTHY, Divya, TCambridge Cambridgeshire CB2 1QT (GB)
(74) Representative: Gibson, Mark
(86) International application number: PCT/GB2011/050662
(87) International publication number: WO 2011/121361

(56) References cited:
- WO-A2-2005/052592
- WO-A2-2008/090319

## Description

### FIELD OF THE INVENTION

The invention relates to a method of diagnosing or monitoring schizophrenia or other psychotic disorder.

### BACKGROUND OF THE INVENTION

Schizophrenia is a psychiatric diagnosis that describes a mental disorder characterized by abnormalities in the perception or expression of reality. It most commonly manifests as auditory hallucinations, paranoid or bizarre delusions, or disorganized speech and thinking with significant social or occupational dysfunction. Onset of symptoms typically occurs in young adulthood, with approximately 0.4-0.6% of the population affected. Diagnosis is based on the patient's self-reported experiences and observed behavior. No laboratory test for schizophrenia currently exists.

Studies suggest that genetics, early environment, neurobiology, psychological and social processes are important contributory factors, and some recreational and prescription drugs appear to cause or worsen symptoms. Current psychiatric research is focused on the role of neurobiology, but no single organic cause has been found. Due to the many possible combinations of symptoms, there is debate about whether the diagnosis represents a single disorder or a number of discrete syndromes.

The disorder is thought to mainly affect cognition, but it also usually contributes to chronic problems with behavior and emotion. People with schizophrenia are likely to have additional (comorbid) conditions, including major depression and anxiety disorders. The lifetime occurrence of substance abuse is around 40%. Social problems, such as long-term unemployment, poverty and homelessness, are common. Furthermore, the average life expectancy of people with the disorder is 10 to 12 years less than those without, due to increased physical health problems and a higher suicide rate.

An important utility of biomarkers for psychotic disorders is their response to medication. Administration of antipsychotics remains a subjective process, relying solely on the experience of clinicians. Furthermore, the development of antipsychotic drugs has been based on chance findings often with little relation to the background driving the observations.

Schizophrenia is treated primarily with antipsychotic medications which are also referred to as neuroleptic drugs or neuroleptics. Newer antipsychotic agents such as clozapine, olanzapine, quetiapine or risperidone are thought to be more effective in improving negative symptoms of psychotic disorders than older medication like chlorpromazine. Furthermore, they induce less extrapyramidal side effects (EPS) which are movement disorders resulting from antipsychotic treatment.

The history of neuroleptics dates back to the late 19th century. The flourishing dye industry catalyzed development of new chemicals that lay the background to modern day atypical antipsychotics. Developments in anti-malaria, antihistamine and anaesthetic compounds also produced various neuroleptics. The common phenomenon to all these processes is a fundamental lack of understanding of the biological mechanisms and pathways that these drugs affect, apart from the observation that they prominently block D2 receptors in the striatum.

There is therefore a pressing need for objective molecular readouts that can diagnose schizophrenia or other psychotic disorders and furthermore indicate whether a patient is responding to medication, as well as for predicting prognosis.

International patent application WO2005/052592 discloses the use of agouti-related protein (AgRP, ART) as a biomarker for Alzheimer.

WO2008/090319 discloses the use of biomarkers such as apolipoprotein A2 as biomarker for schizophrenia.

### SUMMARY OF THE INVENTION

The invention in its broadest form is defined by independent claims 1, 4, 6, 7 and 15:
1. The in vitro use of agouti-related protein, or a fragment greater than 4 amino acids in length thereof, as a biomarker for schizophrenia.
4. The in vitro use of each of the following biomarkers: agouti-related protein, secretagogin, proopiomelanocortin (POMC) or a POMC-derived peptide such as proACTH, ACTH-13, ACTH-8 and ACTH-4.5, neurophysin 2-derived peptides, such as neurophysin 2, vasopressin and copeptin, prohormone convertase 1 (PC1) and growth hormone, or fragments of said biomarkers which are greater than 4 amino acids in length thereof, as a specific panel of biomarkers for schizophrenia.
6. A method of diagnosing schizophrenia or other psychotic disorder, or predisposition in an individual thereto, comprising:
   (a) obtaining a biological sample from an individual;
   (b) quantifying the amounts of the biomarkers defined in any of claims 1 to 5, whereby at least the biomarker of claim 1 is quantified;
   (c) comparing the amounts of the biomarkers in the biological sample with the amounts present in a normal control biological sample from a normal subject, such that a difference in the level of the biomarkers in the biological sample is indicative of schizophrenia; and wherein the biological sample is cerebrospinal fluid, whole blood, blood serum or plasma or an extract or purification therefrom, or dilution thereof.
7. A method of monitoring efficacy of a therapy in a subject having, suspected of having schizophrenia, comprising detecting and/or quantifying, in a sample from said subject, the biomarkers as defined in any of claims 1 to 5, whereby at least the biomarker of claim 1 is detected and/or quantified.
15. Use of a kit for monitoring or diagnosing schizophrenia, comprising a biosensor capable of detecting and/or quantifying the biomarkers as defined in any of claims 1 to 5, whereby at least the biomarker of claim 1 is detected and/or quantified.

Preferred embodiments are defined dependent claims 2, 3, 5 and 8-14.

A further aspect of the disclosure provides ligands, such as naturally occurring or chemically synthesised compounds, capable of specific binding to the peptide biomarker. A ligand according to the disclosure may comprise a peptide, an antibody or a fragment thereof, or an aptamer or oligonucleotide, capable of specific binding to the peptide biomarker. The antibody can be a monoclonal antibody or a fragment thereof capable of specific binding to the peptide biomarker. A ligand according to the disclosure may be labelled with a detectable marker, such as a luminescent, fluorescent or radioactive marker; alternatively or additionally a ligand according to the invention may be labelled with an affinity tag, e.g. a biotin, avidin, streptavidin or His (e.g. hexa-His) tag.

A biosensor according to the disclosure may comprise the peptide biomarker or a structural/shape mimic thereof capable of specific binding to an antibody against the peptide biomarker. Also provided is an array comprising a ligand or mimic as described herein.

Also provided by the disclosure is the use of one or more ligands as described herein, which may be naturally occurring or chemically synthesised, and is suitably a peptide, antibody or fragment thereof, aptamer or oligonucleotide, or the use of a biosensor of the disclosure, or an array of the disclosure, or a kit of the disclosure to detect and/or quantify the peptide. In these uses, the detection and/or quantification can be performed on a biological sample such as from the group consisting of CSF, whole blood, blood serum, plasma, urine, saliva, or other bodily fluid, breath, e.g. as condensed breath, or an extract or purification therefrom, or dilution thereof.

Diagnostic or monitoring kits are provided for performing methods of the disclosure. Such kits will suitably comprise a ligand according to the disclosure, for detection and/or quantification of the peptide biomarker, and/or a biosensor, and/or an array as described herein, optionally together with instructions for use of the kit.

A further aspect of the disclosure is a kit for monitoring or diagnosing schizophrenia or other psychotic disorder, comprising a biosensor capable of detecting and/or quantifying one or more of the first peptide biomarkers as defined herein.

A further aspect of the disclosure is a kit for monitoring or diagnosing schizophrenia or other psychotic disorder, comprising a biosensor capable of detecting and/or quantifying two or more of the second peptide biomarkers as defined herein.

Biomarkers for schizophrenia or other psychotic disorder are essential targets for discovery of novel targets and drug molecules that retard or halt progression of the disorder. As the level of the peptide biomarker is indicative of disorder and of drug response, the biomarker is useful for identification of novel therapeutic compounds in *in vitro* and/or *in vivo* assays. Biomarkers of the invention can be employed in methods for screening for compounds that modulate the activity of the peptide.

Thus, in a further aspect of the disclosure, there is provided the use of a ligand, as described, which can be a peptide, antibody or fragment thereof or aptamer or oligonucleotide according to the disclosure, or the use of a biosensor according to the disclosure, or an array according to the disclosure, or a kit according to the disclosure, to identify a substance capable of promoting and/or of suppressing the generation of the biomarker.

Also there is provided a method of identifying a substance capable of promoting or suppressing the generation of the peptide in a subject, comprising administering a test substance to a subject animal and detecting and/or quantifying the level of the peptide biomarker present in a test sample from the subject.

### BRIEF DESCRIPTION OF THE FIGURES

**Figure 1** describes Western blot analysis of *post-mortem* pituitaries from schizophrenia and control subjects for the expression of PC1 and POMC-derived peptides.
**Figure 2** describes parallel changes in all POMC-derived peptides. a) Correlation of POMC-related peptides across control and SCZ pituitaries (samples indicated on X-axis). b) The expression levels of POMC and the POMC-derived peptides displayed as a histogram.
**Figure 3** describes 2D-DIGE analysis of *post-mortem* pituitaries from schizophrenia and control subjects for expression of proteins.

### DETAILED DESCRIPTION OF THE INVENTION

The invention in its broadest form is defined by independent claims 1, 4, 6, 7 and 15, preferred embodiments are defined dependent claims 2, 3, 5 and 8-14.
1. The in vitro use of agouti-related protein, or a fragment greater than 4 amino acids in length thereof, as a biomarker for schizophrenia.
2. The in vitro use as defined in claim 1, which additionally comprises one or more further biomarkers for schizophrenia selected from: prohormone convertase 1 (PC1), placental growth factor (PLGF), proopiomelanocortin (POMC) or a POMC-derived peptide such as proACTH, ACTH-13, ACTH-8 and ACTH-4.5, secretagogin, growth hormone, prolactin, apolipoprotein AI, apolipoprotein AII, apolipoprotein CIII, alpha-2-HS-glycoprotein, haptoglobin related protein, HBEGF and neurophysin 2-derived peptides such as neurophysin 2, vasopressin and copeptin, such as prohormone convertase 1 (PC1), or fragments of said further biomarkers which are greater than 4 amino acids in length thereof.
3. The in vitro use as defined in claim 2, wherein the further biomarker for schizophrenia is selected from: proopiomelanocortin (POMC) or a POMC-derived peptide such as proACTH, ACTH-13, ACTH-8 and ACTH-4.5, secretagogin, growth hormone and neurophysin 2-derived peptides such as neurophysin 2, vasopressin and copeptin, or fragments of said further biomarkers which are greater than 4 amino acids in length thereof.
4. The in vitro use of each of the following biomarkers: agouti-related protein, secretagogin, proopiomelanocortin (POMC) or a POMC-derived peptide such as proACTH, ACTH-13, ACTH-8 and ACTH-4.5, neurophysin 2-derived peptides, such as neurophysin 2, vasopressin and copeptin, prohormone convertase 1 (PC1) and growth hormone, or fragments of said biomarkers which are greater than 4 amino acids in length thereof, as a specific panel of biomarkers for schizophrenia.
5. The in vitro use as defined in claim 4, wherein the panel additionally comprises each of the following biomarkers for schizophrenia: placental growth factor (PLGF), prolactin, apolipoprotein AI, apolipoprotein AII, apolipoprotein CIII, alpha-2-HS-glycoprotein, haptoglobin related protein and HB-EGF, or fragments of said biomarkers which are greater than 4 amino acids in length thereof.
6. A method of diagnosing schizophrenia or other psychotic disorder, or predisposition in an individual thereto, comprising:
   (a) obtaining a biological sample from an individual;
   (b) quantifying the amounts of the biomarkers defined in any of claims 1 to 5, whereby at least the biomarker of claim 1 is quantified;
   (c) comparing the amounts of the biomarkers in the biological sample with the amounts present in a normal control biological sample from a normal subject, such that a difference in the level of the biomarkers in the biological sample is indicative of schizophrenia; and wherein the biological sample is cerebrospinal fluid, whole blood, blood serum or plasma or an extract or purification therefrom, or dilution thereof.
7. A method of monitoring efficacy of a therapy in a subject having, suspected of having schizophrenia, comprising detecting and/or quantifying, in a sample from said subject, the biomarkers as defined in any of claims 1 to 5, whereby at least the biomarker of claim 1 is detected and/or quantified.
8. A method as defined in claim 6 or claim 7, which is conducted on samples taken on two or more occasions from a test subject.
9. A method as defined in any of claims 6 to 8, further comprising comparing the level of the biomarker present in samples taken on two or more occasions.
10. A method as defined in any of claims 6 to 9, comprising comparing the amount of the biomarker present in said test sample with one or more controls such as comparing the amount of the biomarker in a test sample with the amount of the biomarker present in a sample from a normal subject.
11. A method as defined in any of claims 6 to 10, wherein quantifying is performed by measuring the concentration of the biomarker in the or each sample.
12. A method as defined in any of claims 6 to 11, wherein detecting and/or quantifying is performed by one or more methods selected from SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC or other LC or LC-MS-based technique.
13. A method as defined in any of claims 6 to 12, wherein detecting and/or quantifying is performed using an immunological method, or biosensor or a microanalytical, microengineered, microseparation or immunochromatography system.
14. A method as defined in any of claims 6 to 13, wherein the biological sample is blood serum or an extract or purification therefrom, or dilution thereof.
15. Use of a kit for monitoring or diagnosing schizophrenia, comprising a biosensor capable of detecting and/or quantifying the biomarkers as defined in any of claims 1 to 5, whereby at least the biomarker of claim 1 is detected and/or quantified.

Throughout the disclosed invention at least agouti-related protein is detected or quantified as biomarker for schizophrenia. Other psychotic disorders are mentioned for reference only.

Data is presented herein which demonstrates that this specific panel of biomarkers were found to be significantly altered in *post-mortem* pituitaries from schizophrenia subjects when compared to controls.

The term "biomarker" means a distinctive biological or biologically derived indicator of a process, event, or condition. Peptide biomarkers can be used in methods of diagnosis, e.g. clinical screening, and prognosis assessment and in monitoring the results of therapy, identifying patients most likely to respond to a particular therapeutic treatment, drug screening and development. Biomarkers and uses thereof are valuable for identification of new drug treatments and for discovery of new targets for drug treatment.

As used herein, the term "biosensor" means anything capable of detecting the presence of the biomarker. Examples of biosensors are described herein.

References herein to "other psychotic disorder" relate to any appropriate psychotic disorder according to DSM-IV Diagnostic and Statistical Manual of Mental Disorders, 4th edition, American Psychiatric Assoc, Washington, D.C., 2000. In one particular embodiment, the other psychotic disorder is a psychotic disorder related to schizophrenia. Examples of psychotic disorders related to schizophrenia include brief psychotic disorder delusional disorder, psychotic disorder due to a general medical condition, schizoeffective disorder, schizophreniform disorder, and substance-induced psychotic disorder.

In one embodiment, one or more of the biomarkers defined hereinbefore may be replaced by a molecule, or a measurable fragment of the molecule, found upstream or downstream of the biomarker in a biological pathway.

Biosensors according to the invention may comprise a ligand or ligands, as described herein, capable of specific binding to the peptide biomarker. Such biosensors are useful in detecting and/or quantifying a peptide of the invention.

Diagnostic kits for the diagnosis and monitoring of schizophrenia or other psychotic disorder are described herein. In one embodiment, the kits additionally contain a biosensor capable of detecting and/or quantifying a peptide biomarker.

Monitoring methods of the invention can be used to monitor onset, progression, stabilisation, amelioration and/or remission.

In methods of diagnosing or monitoring according to the invention, detecting and/or quantifying the peptide biomarker in a biological sample from a test subject may be performed on two or more occasions. Comparisons may be made between the level of biomarker in samples taken on two or more occasions. Assessment of any change in the level of the peptide biomarker in samples taken on two or more occasions may be performed. Modulation of the peptide biomarker level is useful as an indicator of the state of schizophrenia or other psychotic disorder or predisposition thereto. An increase in the level of the biomarker, over time is indicative of onset or progression, i.e. worsening of this disorder, whereas a decrease in the level of the peptide biomarker indicates amelioration or remission of the disorder, or vice versa.

A method of diagnosis of or monitoring according to the invention may comprise quantifying the peptide biomarker in a test biological sample from a test subject and comparing the level of the peptide present in said test sample with one or more controls.

The control used in a method of the invention can be one or more control(s) selected from the group consisting of: the level of biomarker peptide found in a normal control sample from a normal subject, a normal biomarker peptide level; a normal biomarker peptide range, the level in a sample from a subject with schizophrenia or other psychotic disorder, or a diagnosed predisposition thereto; schizophrenia or other psychotic disorder biomarker peptide level, or schizophrenia or other psychotic disorder biomarker peptide range.

In one embodiment, there is provided a method of diagnosing schizophrenia or other psychotic disorder, or predisposition thereto, which comprises:
(a) quantifying the amount of the peptide biomarker in a test biological sample; and
(b) comparing the amount of said peptide in said test sample with the amount present in a normal control biological sample from a normal subject.

For biomarkers which are increased in patients with schizophrenia or other psychotic disorder, a higher level of the peptide biomarker in the test sample relative to the level in the normal control is indicative of the presence of schizophrenia or other psychotic disorder, or predisposition thereto, and an equivalent or lower level of the peptide in the test sample relative to the normal control is indicative of absence of schizophrenia or other psychotic disorder and/or absence of a predisposition thereto. For biomarkers which are decreased in patients with schizophrenia or other psychotic disorder, a lower level of the peptide biomarker in the test sample relative to the level in the normal control is indicative of the presence of schizophrenia or other psychotic disorder, or predisposition thereto, and an equivalent or lower level of the peptide in the test sample relative to the normal control is indicative of absence of schizophrenia or other psychotic disorder and/or absence of a predisposition thereto.

The term "diagnosis" as used herein encompasses identification, confirmation, and/or characterisation of schizophrenia or other psychotic disorder, or predisposition thereto. By predisposition it is meant that a subject does not currently present with the disorder, but is liable to be affected by the disorder in time. Methods of monitoring and of diagnosis according to the invention are useful to confirm the existence of a disorder, or predisposition thereto; to monitor development of the disorder by assessing onset and progression, or to assess amelioration or regression of the disorder. Methods of monitoring and of diagnosis are also useful in methods for assessment of clinical screening, prognosis, choice of therapy, evaluation of therapeutic benefit, i.e. for drug screening and drug development.

Efficient diagnosis and monitoring methods provide very powerful "patient solutions" with the potential for improved prognosis, by establishing the correct diagnosis, allowing rapid identification of the most appropriate treatment (thus lessening unnecessary exposure to harmful drug side effects), reducing "downtime" and relapse rates.

Also provided is a method of monitoring efficacy of a therapy for schizophrenia or other psychotic disorder in a subject having such a disorder, suspected of having such a disorder, or of being predisposed thereto, comprising detecting and/or quantifying the peptide present in a biological sample from said subject. In monitoring methods, test samples may be taken on two or more occasions. The method may further comprise comparing the level of the biomarker(s) present in the test sample with one or more control(s) and/or with one or more previous test sample(s) taken earlier from the same test subject, e.g. prior to commencement of therapy, and/or from the same test subject at an earlier stage of therapy. The method may comprise detecting a change in the level of the biomarker(s) in test samples taken on different occasions.

The invention provides a method for monitoring efficacy of therapy for schizophrenia or other psychotic disorder in a subject, comprising:
(a) quantifying the amount of the peptide biomarker; and
(b) comparing the amount of said peptide in said test sample with the amount present in one or more control(s) and/or one or more previous test sample(s) taken at an earlier time from the same test subject.

For biomarkers which are increased in patients with schizophrenia or other psychotic disorder, a decrease in the level of the peptide biomarker in the test sample relative to the level in a previous test sample taken earlier from the same test subject is indicative of a beneficial effect, e.g. stabilisation or improvement, of said therapy on the disorder, suspected disorder or predisposition thereto. For biomarkers which are decreased in patients with schizophrenia or other psychotic disorder, an increase in the level of the peptide biomarker in the test sample relative to the level in a previous test sample taken earlier from the same test subject is indicative of a beneficial effect, e.g. stabilisation or improvement, of said therapy on the disorder, suspected disorder or predisposition thereto.

Methods for monitoring efficacy of a therapy can be used to monitor the therapeutic effectiveness of existing therapies and new therapies in human subjects and in non-human animals (e.g. in animal models). These monitoring methods can be incorporated into screens for new drug substances and combinations of substances.

Suitably, the time elapsed between taking samples from a subject undergoing diagnosis or monitoring will be 3 days, 5 days, a week, two weeks, a month, 2 months, 3 months, 6 or 12 months. Samples may be taken prior to and/or during and/or following an anti-psychotic therapy. Samples can be taken at intervals over the remaining life, or a part thereof, of a subject.

The term "detecting" as used herein means confirming the presence of the peptide biomarker present in the sample. Quantifying the amount of the biomarker present in a sample may include determining the concentration of the peptide biomarker present in the sample. Detecting and/or quantifying may be performed directly on the sample, or indirectly on an extract therefrom, or on a dilution thereof.

In alternative aspects of the invention, the presence of the peptide biomarker is assessed by detecting and/or quantifying antibody or fragments thereof capable of specific binding to the biomarker that are generated by the subject's body in response to the peptide and thus are present in a biological sample from a subject having schizophrenia or other psychotic disorder or a predisposition thereto.

Detecting and/or quantifying can be performed by any method suitable to identify the presence and/or amount of a specific protein in a biological sample from a patient or a purification or extract of a biological sample or a dilution thereof. In methods of the invention, quantifying may be performed by measuring the concentration of the peptide biomarker in the sample or samples. Biological samples that may be tested in a method of the invention include cerebrospinal fluid (CSF), whole blood, blood serum, plasma, urine, saliva, or other bodily fluid (stool, tear fluid, synovial fluid, sputum), breath, e.g. as condensed breath, or an extract or purification therefrom, or dilution thereof. Biological samples also include tissue homogenates, tissue sections and biopsy specimens from a live subject, or taken *post-mortem.* The samples can be prepared, for example where appropriate diluted or concentrated, and stored in the usual manner.

Detection and/or quantification of peptide biomarkers may be performed by detection of the peptide biomarker or of a fragment thereof, e.g. a fragment with C-terminal truncation, or with N-terminal truncation. Fragments are suitably greater than 4 amino acids in length, for example 5, 6, 7, 8, 9, 10, 11, 12, 13, 14, 15, 16, 17, 18, 19, or 20 amino acids in length.

The biomarker may be directly detected, e.g. by SELDI or MALDI-TOF. Alternatively, the biomarker may be detected directly or indirectly via interaction with a ligand or ligands such as an antibody or a biomarker-binding fragment thereof, or other peptide, or ligand, e.g. aptamer, or oligonucleotide, capable of specifically binding the biomarker. The ligand may possess a detectable label, such as a luminescent, fluorescent or radioactive label, and/or an affinity tag.

For example, detecting and/or quantifying can be performed by one or more method(s) selected from the group consisting of: SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC and other LC or LC MS-based techniques. Appropriate LC MS techniques include ICAT® (Applied Biosystems, CA, USA), or iTRAQ® (Applied Biosystems, CA, USA). Liquid chromatography (e.g. high pressure liquid chromatography (HPLC) or low pressure liquid chromatography (LPLC)), thinlayer chromatography, NMR (nuclear magnetic resonance) spectroscopy could also be used.

Methods of diagnosing or monitoring according to the invention may comprise analysing a sample of cerebrospinal fluid (CSF) by SELDI TOF or MALDI TOF to detect the presence or level of the peptide biomarker. These methods are also suitable for clinical screening, prognosis, monitoring the results of therapy, identifying patients most likely to respond to a particular therapeutic treatment, for drug screening and development, and identification of new targets for drug treatment.

Detecting and/or quantifying the peptide biomarkers may be performed using an immunological method, involving an antibody, or a fragment thereof capable of specific binding to the peptide biomarker. Suitable immunological methods include sandwich immunoassays, such as sandwich ELISA, in which the detection of the peptide biomarkers is performed using two antibodies which recognize different epitopes on a peptide biomarker; radioimmunoassays (RIA), direct, indirect or competitive enzyme linked immunosorbent assays (ELISA), enzyme immunoassays (EIA), Fluorescence immunoassays (FIA), western blotting, immunoprecipitation and any particle-based immunoassay (e.g. using gold, silver, or latex particles, magnetic particles, or Q-dots). Immunological methods may be performed, for example, in microtitre plate or strip format.

Immunological methods in accordance with the invention may be based, for example, on any of the following methods.

Immunoprecipitation is the simplest immunoassay method; this measures the quantity of precipitate, which forms after the reagent antibody has incubated with the sample and reacted with the target antigen present therein to form an insoluble aggregate. Immunoprecipitation reactions may be qualitative or quantitative.

In particle immunoassays, several antibodies are linked to the particle, and the particle is able to bind many antigen molecules simultaneously. This greatly accelerates the speed of the visible reaction. This allows rapid and sensitive detection of the biomarker.

In immunonephelometry, the interaction of an antibody and target antigen on the biomarker results in the formation of immune complexes that are too small to precipitate. However, these complexes will scatter incident light and this can be measured using a nephelometer. The antigen, i.e. biomarker, concentration can be determined within minutes of the reaction.

Radioimmunoassay (RIA) methods employ radioactive isotopes such as I¹²⁵ to label either the antigen or antibody. The isotope used emits gamma rays, which are usually measured following removal of unbound (free) radiolabel. The major advantages of RIA, compared with other immunoassays, are higher sensitivity, easy signal detection, and well-established, rapid assays. The major disadvantages are the health and safety risks posed by the use of radiation and the time and expense associated with maintaining a licensed radiation safety and disposal program. For this reason, RIA has been largely replaced in routine clinical laboratory practice by enzyme immunoassays.

Enzyme (EIA) immunoassays were developed as an alternative to radioimmunoassays (RIA). These methods use an enzyme to label either the antibody or target antigen. The sensitivity of EIA approaches that for RIA, without the danger posed by radioactive isotopes. One of the most widely used EIA methods for detection is the enzyme-linked immunosorbent assay (ELISA). ELISA methods may use two antibodies one of which is specific for the target antigen and the other of which is coupled to an enzyme, addition of the substrate for the enzyme results in production of a chemiluminescent or fluorescent signal.

Fluorescent immunoassay (FIA) refers to immunoassays which utilize a fluorescent label or an enzyme label which acts on the substrate to form a fluorescent product. Fluorescent measurements are inherently more sensitive than colorimetric (spectrophotometric) measurements. Therefore, FIA methods have greater analytical sensitivity than EIA methods, which employ absorbance (optical density) measurement.

Chemiluminescent immunoassays utilize a chemiluminescent label, which produces light when excited by chemical energy; the emissions are measured using a light detector.

Immunological methods according to the invention can thus be performed using well-known methods. Any direct (e.g., using a sensor chip) or indirect procedure may be used in the detection of peptide biomarkers of the invention.

The Biotin-Avidin or Biotin-Streptavidin systems are generic labelling systems that can be adapted for use in immunological methods of the invention. One binding partner (hapten, antigen, ligand, aptamer, antibody, enzyme etc) is labelled with biotin and the other partner (surface, e.g. well, bead, sensor etc) is labelled with avidin or streptavidin. This is conventional technology for immunoassays, gene probe assays and (bio)sensors, but is an indirect immobilisation route rather than a direct one. For example a biotinylated ligand (e.g. antibody or aptamer) specific for a peptide biomarker of the invention may be immobilised on an avidin or streptavidin surface, the immobilised ligand may then be exposed to a sample containing or suspected of containing the peptide biomarker in order to detect and/or quantify a peptide biomarker of the invention. Detection and/or quantification of the immobilised antigen may then be performed by an immunological method as described herein.

The term "antibody" as used herein includes, but is not limited to: polyclonal, monoclonal, bispecific, humanised or chimeric antibodies, single chain antibodies, Fab fragments and F(ab')₂ fragments, fragments produced by a Fab expression library, anti-idiotypic (anti-Id) antibodies and epitope-binding fragments of any of the above. The term "antibody" as used herein also refers to immunoglobulin molecules and immunologically-active portions of immunoglobulin molecules, i.e., molecules that contain an antigen binding site that specifically binds an antigen. The immunoglobulin molecules of the invention can be of any class (e. g., IgG, IgE, IgM, IgD and IgA) or subclass of immunoglobulin molecule.

The identification of key biomarkers specific to a disease is central to integration of diagnostic procedures and therapeutic regimes. Using predictive biomarkers appropriate diagnostic tools such as biosensors can be developed, accordingly, in methods and uses of the invention, detecting and quantifying can be performed using a biosensor, microanalytical system, microengineered system, microseparation system, immunochromatography system or other suitable analytical devices. The biosensor may incorporate an immunological method for detection of the biomarker(s), electrical, thermal, magnetic, optical (e.g. hologram) or acoustic technologies. Using such biosensors, it is possible to detect the target biomarker(s) at the anticipated concentrations found in biological samples.

Thus, according to a further aspect of the invention there is provided an apparatus for diagnosing or monitoring schizophrenia or other psychotic disorder which comprises a biosensor, microanalytical, microengineered, microseparation and/or immunochromatography system configured to detect and/or quantify any of the biomarkers defined herein.

The biomarker(s) of the invention can be detected using a biosensor incorporating technologies based on "smart" holograms, or high frequency acoustic systems, such systems are particularly amenable to "bar code" or array configurations.

In smart hologram sensors (Smart Holograms Ltd, Cambridge, UK), a holographic image is stored in a thin polymer film that is sensitised to react specifically with the biomarker. On exposure, the biomarker reacts with the polymer leading to an alteration in the image displayed by the hologram. The test result read-out can be a change in the optical brightness, image, colour and/or position of the image. For qualitative and semi-quantitative applications, a sensor hologram can be read by eye, thus removing the need for detection equipment. A simple colour sensor can be used to read the signal when quantitative measurements are required. Opacity or colour of the sample does not interfere with operation of the sensor. The format of the sensor allows multiplexing for simultaneous detection of several substances. Reversible and irreversible sensors can be designed to meet different requirements, and continuous monitoring of a particular biomarker of interest is feasible.

Suitably, biosensors for detection of one or more biomarkers of the invention combine biomolecular recognition with appropriate means to convert detection of the presence, or quantitation, of the biomarker in the sample into a signal. Biosensors can be adapted for "alternate site" diagnostic testing, e.g. in the ward, outpatients' department, surgery, home, field and workplace.

Biosensors to detect one or more biomarkers of the invention include acoustic, plasmon resonance, holographic and microengineered sensors. Imprinted recognition elements, thin film transistor technology, magnetic acoustic resonator devices and other novel acousto-electrical systems may be employed in biosensors for detection of the one or more biomarkers of the invention.

Methods involving detection and/or quantification of one or more peptide biomarkers of the invention can be performed on bench-top instruments, or can be incorporated onto disposable, diagnostic or monitoring platforms that can be used in a non-laboratory environment, e.g. in the physician's office or at the patient's bedside. Suitable biosensors for performing methods of the invention include "credit" cards with optical or acoustic readers. Biosensors can be configured to allow the data collected to be electronically transmitted to the physician for interpretation and thus can form the basis for e-neuromedicine.

Any suitable animal may be used as a subject non-human animal, for example a non-human primate, horse, cow, pig, goat, sheep, dog, cat, fish, rodent, e.g. guinea pig, rat or mouse; insect (e.g. *Drosophila),* amphibian (e.g. *Xenopus*) or *C. elegans.*

The test substance can be a known chemical or pharmaceutical substance, such as, but not limited to, an anti-psychotic disorder therapeutic; or the test substance can be novel synthetic or natural chemical entity, or a combination of two or more of the aforesaid substances.

There is provided a method of identifying a substance capable of promoting or suppressing the generation of the peptide biomarker in a subject, comprising exposing a test cell to a test substance and monitoring the level of the peptide biomarker within said test cell, or secreted by said test cell.

The test cell could be prokaryotic, however a eukaryotic cell will suitably be employed in cell-based testing methods. Suitably, the eukaryotic cell is a yeast cell, insect cell, *Drosophila* cell, amphibian cell (e.g. from *Xenopus*), *C. elegans* cell or is a cell of human, non-human primate, equine, bovine, porcine, caprine, ovine, canine, feline, piscine, rodent or murine origin.

In methods for identifying substances of potential therapeutic use, non-human animals or cells can be used that are capable of expressing the peptide.

Screening methods also encompass a method of identifying a ligand capable of binding to the peptide biomarker according to the invention, comprising incubating a test substance in the presence of the peptide biomarker in conditions appropriate for binding, and detecting and/or quantifying binding of the peptide to said test substance.

High-throughput screening technologies based on the biomarker, uses and methods of the invention, e.g. configured in an array format, are suitable to monitor biomarker signatures for the identification of potentially useful therapeutic compounds, e.g. ligands such as natural compounds, synthetic chemical compounds (e.g. from combinatorial libraries), peptides, monoclonal or polyclonal antibodies or fragments thereof, which may be capable of binding the biomarker.

Methods of the invention can be performed in array format, e.g. on a chip, or as a multiwell array. Methods can be adapted into platforms for single tests, or multiple identical or multiple non-identical tests, and can be performed in high throughput format. Methods of the invention may comprise performing one or more additional, different tests to confirm or exclude diagnosis, and/or to further characterise a condition.

The invention further provides a substance, e.g. a ligand, identified or identifiable by an identification or screening method or use of the invention. Such substances may be capable of inhibiting, directly or indirectly, the activity of the peptide biomarker, or of suppressing generation of the peptide biomarker. The term "substances" includes substances that do not directly bind the peptide biomarker and directly modulate a function, but instead indirectly modulate a function of the peptide biomarker. Ligands are also included in the term substances; ligands of the invention (e.g. a natural or synthetic chemical compound, peptide, aptamer, oligonucleotide, antibody or antibody fragment) are capable of binding, suitably specific binding, to the peptide.

The invention further provides a substance according to the invention for use in the treatment of schizophrenia or other psychotic disorder, or predisposition thereto.

Also provided is the use of a substance according to the invention in the treatment of schizophrenia or other psychotic disorder, or predisposition thereto.

Also provided is the use of a substance according to the invention as a medicament.

A kit for diagnosing or monitoring schizophrenia or other psychotic disorder, or predisposition thereto is provided. Suitably a kit according to the invention may contain one or more components selected from the group: a ligand specific for the peptide biomarker or a structural/shape mimic of the peptide biomarker, one or more controls, one or more reagents and one or more consumables; optionally together with instructions for use of the kit in accordance with any of the methods defined herein.

The identification of biomarkers for schizophrenia or other psychotic disorder permits integration of diagnostic procedures and therapeutic regimes. Currently there are significant delays in determining effective treatment and hitherto it has not been possible to perform rapid assessment of drug response. Traditionally, many anti-psychotic therapies have required treatment trials lasting weeks to months for a given therapeutic approach. Detection of a peptide biomarker of the invention can be used to screen subjects prior to their participation in clinical trials. The biomarkers provide the means to indicate therapeutic response, failure to respond, unfavourable side-effect profile, degree of medication compliance and achievement of adequate serum drug levels. The biomarkers may be used to provide warning of adverse drug response. Biomarkers are useful in development of personalized brain therapies, as assessment of response can be used to fine-tune dosage, minimise the number of prescribed medications, reduce the delay in attaining effective therapy and avoid adverse drug reactions. Thus by monitoring a biomarker of the invention, patient care can be tailored precisely to match the needs determined by the disorder and the pharmacogenomic profile of the patient, the biomarker can thus be used to titrate the optimal dose, predict a positive therapeutic response and identify those patients at high risk of severe side effects.

Biomarker-based tests provide a first line assessment of 'new' patients, and provide objective measures for accurate and rapid diagnosis, in a time frame and with precision, not achievable using the current subjective measures.

Furthermore, diagnostic biomarker tests are useful to identify family members or patients at high risk of developing schizophrenia or other psychotic disorder. This permits initiation of appropriate therapy, or preventive measures, e.g. managing risk factors. These approaches are recognised to improve outcome and may prevent overt onset of the disorder.

Biomarker monitoring methods, biosensors and kits are also vital as patient monitoring tools, to enable the physician to determine whether relapse is due to worsening of the disorder, poor patient compliance or substance abuse. If pharmacological treatment is assessed to be inadequate, then therapy can be reinstated or increased; a change in therapy can be given if appropriate. As the biomarkers are sensitive to the state of the disorder, they provide an indication of the impact of drug therapy or of substance abuse.

The following study illustrates the invention.

The multiple technologies of Western blotting (WB), liquid chromatography mass spectrometry (LC-MS^{E}, multiplex immunoassays (HumanMAP®) and two-dimensional difference gel electrophoresis (2D-DIGE) were used to carry out systematic profiling of *post-mortem* pituitaries from schizophrenia and control subjects, obtained from the Stanley Medical Research Institute.

### Example 1: Western Blot Analysis

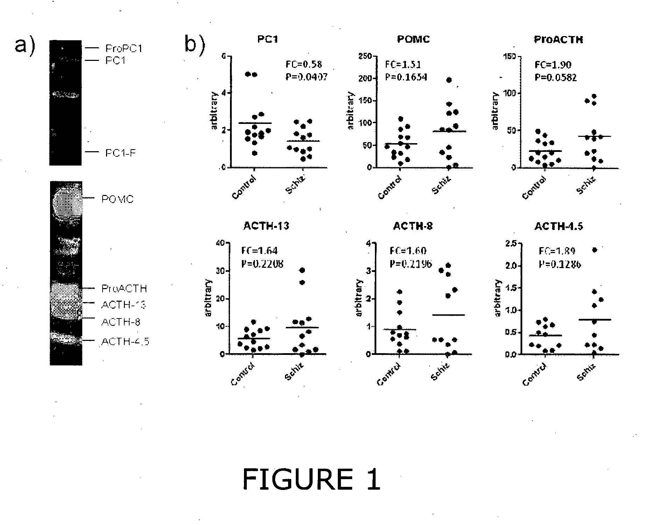

Tissue was prepared and Western blot analysis carried out as described previously (Wang, L. et al (2010) J Proteome Res. 9, 521-527). For example, pituitaries were frozen in liquid nitrogen, pulverized using a mortar and pestle, and the proteins were extracted by boiling in SDS-PAGE sample loading buffer. The proteins were then subjected to Western blot analysis using antibodies against either proopiomelanocortin (POMC) or prohormone convertase 1 (PC1). The results are shown in Figure 1 which describes the Western blot images of PC1, POMC and the POMC-derived peptides proACTH, ACTH-13, ACTH-8 and ACTH-4.5 (Figure 1a). The relative densities of the bands corresponding to the indicated proteins were determined using the Odyssey Infrared Imaging Scanner (LI-COR Bioscience, Lincoln, NE, USA). These were displayed as scatter plots where each spot is the expression level of the indicated molecule in each control and schizophrenia subject (Figure 1b).

The Western blot studies identified decreased expression of PC1 and increased expression of the precursor and 4 peptides derived from POMC (proACTH, ACTH-13, ACTH-8 and ACTH-4.5) in *post-mortem* pituitaries from schizophrenia compared to control subjects. The decreased expression of PC1 may explain accumulation of POMC and the POMC-derived peptides observed in Figure 1.

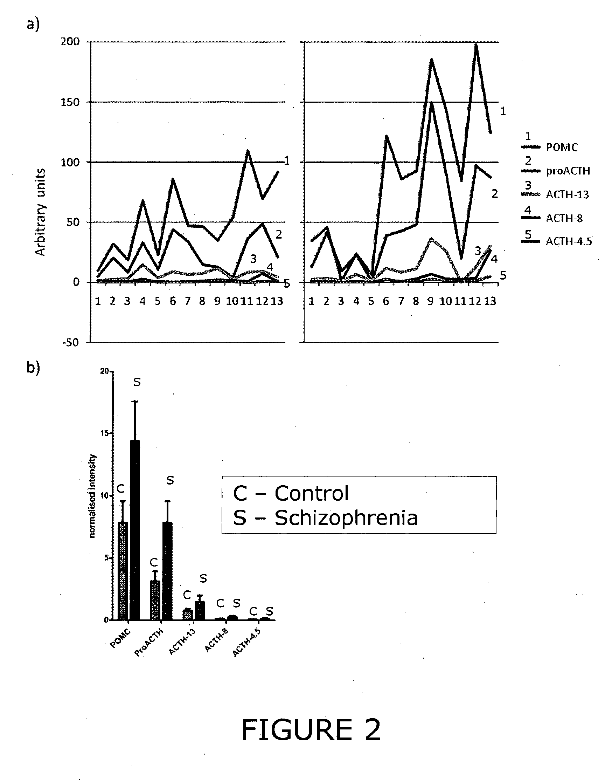

An analysis of POMC and the POMC-derived peptides was conducted at the level of individual pituitaries to identify potential molecular correlations and the results are shown in Figure 2. These results demonstrate that although none of the POMC-related peptides reached significance when considered separately, the expression of these peptides considered together showed a tight correlation (Figure 2a) and the correlated peptides showed greater significance (P=0.0520; Figure 2b).

Thus, the results of the Western blot analysis demonstrate that expression levels of PC1, POMC and the POMC-derived peptides (proACTH, ACTH-13, ACTH-8 and ACTH-4.5) were altered in schizophrenia subjects compared to controls.

### Example 2: LC-MS^{E} Analysis

LC-MS^{E} analysis of soluble and insoluble fractions was carried out as described previously (Levin, Y. et al., (2009) J Proteomics 73, 689-695). To generate the soluble protein fraction, frozen tissue was sonicated in 30mM Tris (pH 8.0), the samples were centrifuged at 13,000g for 30 min and the resulting supernatant stored at -80°C prior to analysis. For the insoluble fraction, the pellets were sonicated in 8M urea, centrifuged as above and the resulting supernatants diluted with 3 volumes of 50mM ammonium bicarbonate (such that the level of urea present was 2M) and stored at -80°C prior to analysis. Analysis of both fractions resulted in the identification of approximately 1300 non-redundant proteins represented by a minimum of two peptides. Of these, approximately 40 proteins were identified that were differentially expressed in pituitaries from schizophrenia compared to control subjects, including hormones, secreted factors, lipid transport proteins, enzymes, histones and structural proteins. Data were normalised using total ion current and processed using PLGS v 2.3 and Elucidator v.3.3. Statistical comparison was carried out using Student's t test. Significance was set at p<0.05. Only the secreted proteins or other proteins likely to be identified in serum as biomarker candidates are indicated (Table 1).

**Table 1: Significant Markers identified by LC-MS^{E} analysis**

| **Protein** | **P Value** | **Fold Change** |
|---|---|---|
| Proopiomelanocortin | 0.080*** | 1.20 |
| Apolipoprotein AI | 0.033 | 1.25 |
| Apolipoprotein AII | 0.022 | -1.46 |
| Alpha-2-HS-Glycoprotein | 0.012 | -1.53 |
| Prolactin | 0.020 | -1.56 |
| Vasopressin-neurophysin 2-copeptin | 0.043 | -1.62 |
| Secretagogin | 0.011 | -1.80 |
| Haptoglobin-related protein | 0.012 | -2.44 |

| | | |
|---|---|---|
| ***indicates this sample was included due to borderline significance and it was detected with other technologies. | | |

The differentially expressed proteins from the LC-MS^{E} analysis were then ranked according to the following parameters to maximize the chances of translation to a robust user friendly format:
1) the novelty of the finding;
2) whether the candidate is known to be secreted; and
3) whether assays currently exist for measurement of the candidates in serum.

The preferred markers from this ranking were secretagogin, neurophysin 2, POMC and POMC-derived peptides (proACTH, ACTH-13, ACTH-8 and ACTH-4.5).

### Example 3: Multiplex Immunoassays

HumanMAP analysis was carried out on soluble extracts of pituitary tissue from schizophrenia and control subjects using the HumanMAP platform in collaboration with Rules Based Medicine as described previously (Cheng, T.M. et al., (2010) Mol Cell Proteomics 9, 510-522). Soluble extracts were prepared by sonication of pulverized frozen pituitaries in 50 mM Tris (pH 7.4) containing protease inhibitors, centrifugation at 13,000g for 20 min and storage of the supernatant at -80°C.

Approximately 10 proteins were identified that were differentially expressed in pituitaries from schizophrenia compared to control subjects. Only the secreted proteins or other proteins likely to be identified in serum as biomarker candidates are indicated (Table 2).

**Table 2: Significant Markers identified in HumanMAP analysis**

| **Protein** | **P Value** | **Fold Change** |
|---|---|---|
| Agouti-related protein | 0.021 | 1.98 |
| Apolipoprotein AII | 0.002 | -1.18 |
| Adrenocorticotrophic hormone (ACTH) | 0.039 | -1.20 |
| Apolipoprotein CIII | 0.010 | -1.27 |
| HB-EGF | 0.002 | -1.47 |
| Placental growth factor (PLGF) | 0.029 | -1.82 |

The differentially expressed proteins from the HumanMAP analysis were then ranked according to the parameters used in Example 2 which led to the identification of agouti-related protein as the preferred marker.

### Example 4: 2D-DIGE Analysis

Soluble and insoluble extracts of pituitary tissue from schizophrenia and control subjects were prepared as described above and analysed by 2D-DIGE as described previously (Knowles, M.R. et al., (2003) Proteomics 3, 1162-1171). Pituitaries were frozen in liquid nitrogen, pulverized using a mortar and pestle and proteins extracted into soluble and insoluble fractions. The proteins were then subjected to 2D-DIGE analysis.

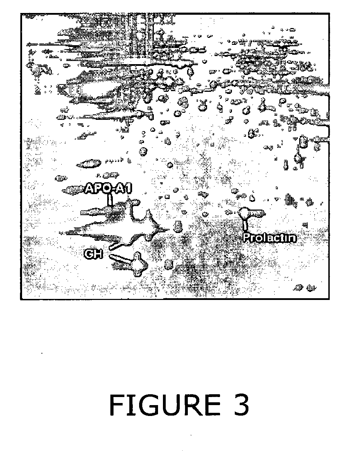

Approximately 30 protein spots were identified which were differentially expressed in pituitaries from schizophrenia compared to control subjects. Only the secreted proteins or other proteins likely to be identified in serum as biomarker candidates are indicated (Figure 3). Consistent with at least one other technology above, this led to identification of apolipoprotein-AI (APO-A1) and Prolactin. In addition, this also resulted in identification of changes in growth hormone (GH). Thus, the results of this analysis demonstrated that these 3 proteins which are known to be secreted also showed differential expression. Adopting the assessment parameters described in Example 2, Growth hormone was selected as the lead candidate from the 3 differentially expressed proteins identified following 2D-DIGE analysis.

## Claims

1. The *in vitro* use of agouti-related protein, or a fragment greater than 4 amino acids in length thereof, as a biomarker for schizophrenia.

2. The *in vitro* use as defined in claim 1, which additionally comprises one or more further biomarkers for schizophrenia selected from: prohormone convertase 1 (PC1), placental growth factor (PLGF), proopiomelanocortin (POMC) or a POMC-derived peptide such as proACTH, ACTH-13, ACTH-8 and ACTH-4.5, secretagogin, growth hormone, prolactin, apolipoprotein AI, apolipoprotein AII, apolipoprotein CIII, alpha-2-HS-glycoprotein, haptoglobin related protein, HB-EGF and neurophysin 2-derived peptides such as neurophysin 2, vasopressin and copeptin, such as prohormone convertase 1 (PC1), or fragments of said further biomarkers which are greater than 4 amino acids in length thereof.

3. The *in vitro* use as defined in claim 2, wherein the further biomarker for schizophrenia is selected from: proopiomelanocortin (POMC) or a POMC-derived peptide such as proACTH, ACTH-13, ACTH-8 and ACTH-4.5, secretagogin, growth hormone and neurophysin 2-derived peptides such as neurophysin 2, vasopressin and copeptin, or fragments of said further biomarkers which are greater than 4 amino acids in length thereof.

4. The *in vitro* use of each of the following biomarkers: agouti-related protein, secretagogin, proopiomelanocortin (POMC) or a POMC-derived peptide such as proACTH, ACTH-13, ACTH-8 and ACTH-4.5, neurophysin 2-derived peptides, such as neurophysin 2, vasopressin and copeptin, prohormone convertase 1 (PC1) and growth hormone, or fragments of said biomarkers which are greater than 4 amino acids in length thereof, as a specific panel of biomarkers for schizophrenia.

5. The *in vitro* use as defined in claim 4, wherein the panel additionally comprises each of the following biomarkers for schizophrenia: placental growth factor (PLGF), prolactin, apolipoprotein AI, apolipoprotein AII, apolipoprotein CIII, alpha-2-HS-glycoprotein, haptoglobin related protein and HB-EGF, or fragments of said biomarkers which are greater than 4 amino acids in length thereof.

6. A method of diagnosing schizophrenia or other psychotic disorder, or predisposition in an individual thereto, comprising:
(a) obtaining a biological sample from an individual;
(b) quantifying the amounts of the biomarkers defined in any of claims 1 to 5; whereby at least the biomarker of claim 1 is quantified;
(c) comparing the amounts of the biomarkers in the biological sample with the amounts present in a normal control biological sample from a normal subject, such that a difference in the level of the biomarkers in the biological sample is indicative of schizophrenia; and
wherein the biological sample is cerebrospinal fluid, whole blood, blood serum or plasma or an extract or purification therefrom, or dilution thereof.

7. A method of monitoring efficacy of a therapy in a subject having, suspected of having schizophrenia, comprising detecting and/or quantifying, in a sample from said subject, the biomarkers as defined in any of claims 1 to 5, whereby at least the biomarker of claim 1 is detected and/or quantified.

8. A method as defined in claim 6 or claim 7, which is conducted on samples taken on two or more occasions from a test subject.

9. A method as defined in any of claims 6 to 8, further comprising comparing the level of the biomarker present in samples taken on two or more occasions.

10. A method as defined in any of claims 6 to 9, comprising comparing the amount of the biomarker present in said test sample with one or more controls such as comparing the amount of the biomarker in a test sample with the amount of the biomarker present in a sample from a normal subject.

11. A method as defined in any of claims 6 to 10, wherein quantifying is performed by measuring the concentration of the biomarker in the or each sample.

12. A method as defined in any of claims 6 to 11, wherein detecting and/or quantifying is performed by one or more methods selected from SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC or other LC or LC-MS-based technique.

13. A method as defined in any of claims 6 to 12, wherein detecting and/or quantifying is performed using an immunological method, or biosensor or a microanalytical, microengineered, microseparation or immunochromatography system.

14. A method as defined in any of claims 6 to 13, wherein the biological sample is blood serum or an extract or purification therefrom, or dilution thereof.

15. Use of a kit for monitoring or diagnosing schizophrenia, comprising a biosensor capable of detecting and/or quantifying the biomarkers as defined in any of claims 1 to 5,
whereby at least the biomarker of claim 1 is detected and/or quantified.

## Patentansprüche

1. *In-vitro*-Verwendung von Agouti-bezogenem Protein oder einem Fragment davon mit einer Länge von mehr als 4 Aminosäuren als Biomarker für Schizophrenie.

2. *In-vitro*-Verwendung nach Anspruch 1, die zusätzlich einen oder mehrere weitere Biomarker für Schizophrenie beinhaltet, ausgewählt aus: Prohormon-Convertase 1 (PC1), plazentarem Wachstumsfaktor (PLGF), Proopiomelanocortin (POMC) oder einem von POMC abgeleiteten Peptid wie proACTH, ACTH-13, ACTH-8 und ACTH-4,5, Secretagogin, Wachstumshormon, Prolactin, Apolipoprotein AI, Apolipoprotein AII, Apolipoprotein CIII, Alpha-2-HS-Glykoprotein, Haptoglobin-bezogenem Protein, HB-EGF und von Neurophysin 2 abgeleiteten Peptiden wie Neurophysin 2, Vasopressin und Copeptin, wie Prohormon-Convertase 1 (PC1), oder Fragmenten der genannten weiteren Biomarker, die eine Länge von mehr als 4 Aminosäuren haben.

3. *In-vitro*-Verwendung nach Anspruch 2, wobei der weitere Biomarker für Schizophrenie ausgewählt ist aus: Proopiomelanocortin (POMC) oder einem von POMC abgeleiteten Derivat wie proACTH, ACTH-13, ACTH-8 und ACTH-4,5, Secretagogin, Wachstumshormon und von Neurophysin II abgeleiteten Peptiden wie Neurophysin II, Vasopressin und Copeptin, oder Fragmenten der genannten weiteren Biomarker, die eine Länge von mehr als 4 Aminosäuren haben.

4. In-vitro-Verwendung von jedem der folgenden Biomarker: Agoutibezogenes Protein, Secretagogin, Proopiomelanocortin (POMC) oder ein von POMC abgeleitetes Peptid wie proACTH, ACTH-13, ACTH-8 und ACTH-4,5, von Neurophysin II abgeleitete Peptide wie Neurophysin II, Vasopressin und Copeptin, Prohormon-Convertase 1 (PC1) und Wachstumshormone oder Fragmente der genannten Biomarker, die eine Länge von mehr als 4 Aminosäuren haben, als spezifisches Panel von Biomarkern für Schizophrenie.

5. *In-vitro*-Verwendung nach Anspruch 4, wobei das Panel zusätzlich jeden der folgenden Biomarker für Schizophrenie beinhaltet: plazentaren Wachstumsfaktor (PLGF), Prolactin, Apolipoprotein AI, Apolipoprotein AII, Apolipoprotein CIII, Alpha-2-HS-Glykoprotein, Haptoglobin-bezogenes Protein und HB-EGF, oder Fragmente der genannten Biomarker, die eine Länge von mehr als 4 Aminosäuren haben.

6. Verfahren zum Diagnostizieren von Schizophrenie oder einer anderen psychotischen Störung oder Prädisposition dafür in einem Individuum, das Folgendes beinhaltet:
(a) Gewinnen einer biologischen Probe von einem Individuum;
(b) Quantifizieren der Mengen der Biomarker gemäß Definition in einem der Ansprüche 1 bis 5, wobei wenigstens der Biomarker von Anspruch 1 quantifiziert wird;
(c) Vergleichen der Mengen der Biomarker in der biologischen Probe mit den in einer normalen biologischen Kontrollprobe von einem normalen Subjekt vorliegenden Mengen, so dass eine Differenz im Spiegel der Biomarker in der biologischen Probe Schizophrenie anzeigt; und
wobei die biologische Probe Rückenmarksflüssigkeit, Vollblut, Blutserum oder -plasma oder ein Extraktions- oder Reinigungsprodukt davon oder ein Verdünnungsprodukt davon enthält.

7. Verfahren zum Überwachen der Wirksamkeit einer Therapie in einem Subjekt, das Schizophrenie hat oder bei dem Schizophrenie vermutet wird, das das Nachweisen und/oder Quantifizieren, in einer Probe von dem genannten Subjekt, der Biomarker gemäß Definition in einem der Ansprüche 1 bis 5 beinhaltet, wobei wenigstens der Biomarker von Anspruch 1 nachgewiesen und/der quantifiziert wird.

8. Verfahren nach Anspruch 6 oder Anspruch 7, das an zwei- oder mehrmalig von einem Testsubjekt genommenen Proben durchgeführt wird.

9. Verfahren nach einem der Ansprüche 6 bis 8, das ferner das Vergleichen des Spiegels des Biomarkers beinhaltet, der in an zwei- oder mehrmalig genommenen Proben vorliegt.

10. Verfahren nach einem der Ansprüche 6 bis 9, das das Vergleichen der Menge des in der genannten Testprobe vorliegenden Biomarkers mit einer oder mehreren Kontrollen wie z.B. das Vergleichen der Menge des Biomarkers in einer Testprobe mit der Menge des in einer Probe von einem normalen Subjekt vorhandenen Biomarkers beinhaltet.

11. Verfahren nach einem der Ansprüche 6 bis 10, wobei das Quantifizieren durch Messen der Konzentration des Biomarkers in der oder jeder Probe durchgeführt wird.

12. Verfahren nach einem der Ansprüche 6 bis 11, wobei das Nachweisen und/oder Quantifizieren mit einem oder mehreren Verfahren durchgeführt wird, die ausgewählt sind aus SELDI (-TOF), MALDI (-TOF), einer Analyse auf der Basis von 1D-Gel, einer Analyse auf der Basis von 2D-Gel, Massenspektrometrie (MS), Umkehrphasen-(RP)-LC, Größenpermeation (Gelfiltration), Ionenaustausch, Affinität, HPLC, UPLC oder einer anderen Technik auf LC- oder LC-MS-Basis.

13. Verfahren nach einem der Ansprüche 6 bis 12, wobei das Nachweisen und/oder Quantifizieren mit einem immunologischen Verfahren oder einem Biosensor oder einem mikroanalytischen, mikrobearbeiteten, Mikrotrenn- oder Immunchromatografiesystem durchgeführt wird.

14. Verfahren nach einem der Ansprüche 6 bis 13, wobei die biologische Probe Blutserum oder ein Extraktions- oder Reinigungsprodukt davon oder ein Verdünnungsprodukt davon ist.

15. Verwendung eines Kit zum Überwachen oder Diagnostizieren von Schizophrenie, der einen Biosensor umfasst, der die Biomarker gemäß Definition in einem der Ansprüche 1 bis 5 nachweisen und/oder quantifizieren kann, wobei wenigstens der Biomarker von Anspruch 1 nachgewiesen und/oder quantifiziert wird.

## Revendications

1. Utilisation *in vitro* de la protéine apparentée à agouti ou d'un fragment de plus de 4 acides aminés de long de celle-ci en tant que biomarqueur de la schizophrénie.

2. Utilisation *in vitro* selon la revendication 1, qui comprend également un ou plusieurs biomarqueurs de la schizophrénie additionnels sélectionnés parmi les suivants : prohormone convertase-1 (PC1), facteur de croissance placentaire (PIGF), pro-opiomélanocortine (POMC) ou peptide dérivé de la POMC comme la proACTH, l'ACTH-13, l'ACTH-8 et l'ACTH-4,5, sécrétagogine, hormone de croissance, prolactine, apolipoprotéine A-I, apolipoprotéine A-II, apolipoprotéine C-III, alpha-2-HS-glycoprotéine, protéine apparentée à l'haptoglobine, HB-EGF et peptides dérivés de la neurophysine II comme la neurophysine II, la vasopressine et la copeptine, comme la prohormone convertase-1 (PC1), ou fragments de plus de 4 acides aminés de long desdits biomarqueurs additionnels.

3. Utilisation *in vitro* selon la revendication 2, dans laquelle le biomarqueur de la schizophrénie additionnel est sélectionné parmi les suivants : pro-opiomélanocortine (POMC) ou peptide dérivé de la POMC comme la proACTH, l'ACTH-13, l'ACTH-8 et l'ACTH-4,5, sécrétagogine, hormone de croissance et peptides dérivés de la neurophysine II comme la neurophysine II, la vasopressine et la copeptine, ou fragments de plus de 4 acides aminés de long desdits biomarqueurs additionnels.

4. Utilisation *in vitro* de chacun des biomarqueurs suivants : protéine apparentée à agouti, sécrétagogine, pro-opiomélanocortine (POMC) ou peptide dérivé de la POMC comme la proACTH, l'ACTH-13, l'ACTH-8 et l'ACTH-4,5, peptides dérivés de la neurophysine II comme la neurophysine II, la vasopressine et la copeptine, prohormone convertase-1 (PC1) et hormone de croissance, ou fragments de plus de 4 acides aminés de long desdits biomarqueurs, en tant que batterie spécifique de biomarqueurs de la schizophrénie.

5. Utilisation *in vitro* selon la revendication 4, dans laquelle la batterie comprend également chacun des biomarqueurs de la schizophrénie suivants : facteur de croissance placentaire (PIGF), prolactine, apolipoprotéine A-I, apolipoprotéine A-II, apolipoprotéine C-III, alpha-2-HS-glycoprotéine, protéine apparentée à l'haptoglobine et HB-EGF, ou fragments de plus de 4 acides aminés de long desdits biomarqueurs.

6. Méthode de diagnostic de la schizophrénie ou d'une autre affection psychotique ou d'une prédisposition à un tel trouble chez un individu, qui comprend :
(a) l'obtention d'un échantillon biologique chez un individu ;
(b) la quantification des quantités des biomarqueurs selon l'une quelconque des revendications 1 à 5, dans laquelle au moins le biomarqueur de la revendication 1 est quantifié ;
(c) la comparaison entre les quantités de biomarqueurs mesurées dans l'échantillon biologique et les quantités présentes dans un échantillon biologique contrôle normal obtenu chez un sujet normal, de sorte qu'une différence dans le taux des biomarqueurs dans l'échantillon biologique est indicatrice de schizophrénie ; et
dans laquelle l'échantillon biologique est le liquide céphalorachidien, le sang entier, le sérum ou le plasma sanguin ou un extrait ou une forme purifiée ou diluée de ceux-ci.

7. Méthode de suivi de l'efficacité d'un traitement chez un sujet atteint ou suspecté d'être atteint de schizophrénie, qui comprend la détection et/ou la quantification, dans un échantillon obtenu chez ledit sujet, des biomarqueurs selon l'une quelconque des revendications 1 à 5, dans laquelle au moins le biomarqueur de la revendication 1 est détecté et/ou quantifié.

8. Méthode selon la revendication 6 ou la revendication 7, qui est effectuée sur des échantillons prélevés à deux occasions ou plus chez un sujet à tester.

9. Méthode selon l'une quelconque des revendications 6 à 8, qui comprend également la comparaison des taux de biomarqueurs présents dans des échantillons prélevés à deux occasions ou plus.

10. Méthode selon l'une quelconque des revendications 6 à 9, qui comprend la comparaison de la quantité de biomarqueur présente dans ledit échantillon à tester et dans un ou plusieurs contrôles, par exemple la comparaison entre la quantité de biomarqueur présente dans un échantillon à tester et la quantité du biomarqueur dans un échantillon obtenu chez un sujet normal.

11. Méthode selon l'une quelconque des revendications 6 à 10, dans laquelle la quantification est effectuée en mesurant la concentration du biomarqueur dans l'échantillon ou dans chaque échantillon.

12. Méthode selon l'une quelconque des revendications 6 à 11, dans laquelle la détection et/ou la quantification est effectuée en utilisant une ou plusieurs techniques sélectionnées parmi les suivantes : SELDI (-TOF), MALDI (-TOF), analyse sur gel 1-D, analyse sur gel 2-D, spectrométrie de masse (SM), CL en phase inverse (PI), chromatographie par perméation de gel (filtration sur gel), chromatographie par échange d'ions, chromatographie d'affinité, CLHP, CLUP ou autre technique reposant sur la CL ou la CL-SM.

13. Méthode selon l'une quelconque des revendications 6 à 12, dans laquelle la détection et/ou la quantification est effectuée en utilisant une méthode immunologique ou un biocapteur ou un système de microanalyse, de micro-mécanique, de microséparation ou d'immunochromatographie.

14. Méthode selon l'une quelconque des revendications 6 à 13, dans laquelle l'échantillon biologique est le sérum sanguin ou un extrait ou une forme purifiée ou diluée de celui-ci.

15. Utilisation d'un kit pour suivre ou diagnostiquer la schizophrénie, qui comprend un biocapteur capable de détecter et/ou de quantifier les biomarqueurs selon l'une quelconque des revendications 1 à 5, dans laquelle au moins le biomarqueur de la revendication 1 est détecté et/ou quantifié.
